(19) Europäisches Patentamt
European Patent Office
Office européen des brevets

(11) Veröffentlichungsnummer: **0 299 286**
**B1**

(12) **EUROPÄISCHE PATENTSCHRIFT**

(45) Veröffentlichungstag der Patentschrift: **02.01.91**

(21) Anmeldenummer: **88110446.7**

(22) Anmeldetag: **30.06.88**

(51) Int. Cl.$^5$: **C 07 C 43/16**, C 07 C 43/166, C 07 C 43/174, C 07 C 43/188, C 07 C 43/162, C 07 C 43/17, C 07 C 41/28

# (54) Verfahren zur Herstellung von Vinylethern.

(30) Priorität: **10.07.87 DE 3722891**

(43) Veröffentlichungstag der Anmeldung:
**18.01.89 Patentblatt 89/03**

(45) Bekanntmachung des Hinweises auf die Patenterteilung:
**02.01.91 Patentblatt 91/01**

(84) Benannte Vertragsstaaten:
**BE CH DE FR GB IT LI NL**

(56) Entgegenhaltungen:
**EP-A-0 217 089**
**DE-C- 560 354**

(73) Patentinhaber: **BASF Aktiengesellschaft**
**Carl-Bosch-Strasse 38**
**D-6700 Ludwigshafen (DE)**

(72) Erfinder: **Hoelderich, Wolfgang**
**Mannheimer Strasse 18 c**
**D-6710 Frankenthal (DE)**
Erfinder: **Goetz, Norbert, Dr.**
**Schoefferstrasse 25**
**D-6520 Worms 1 (DE)**
Erfinder: **Hupfer, Leopold, Dr.**
**Waltershoehe 3**
**D-6701 Friedelsheim (DE)**

**EP 0 299 286 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein Verfahren zur Herstellung von Vinylethern durch Abspaltung von Alkoholen aus Acetalen/Ketalen in Gegenwart von Katalysatoren.

Vinylether werden bei der Herstellung bestimmter Homo- und Copolymerisate eingesetzt, die Anwendung auf den Gebieten der Lack- und Klebstoffherstellung sowie als Hilfsmittel in der Textil- und Lederindustrie finden. Weiterhin dienen Vinylether als wertvolle Zwischenprodukte für organische Synthesen z.B. für Diels-Adler-Reaktionen, für die Herstellung von Glutardialdehyden, γ-Pyran und γ-Picolin sowie Wirkstoffe.

Technisch werden Vinylether nach Reppe aus Acetylen und Alkoholen in Flüssigphase mit Kaliumhydroxid als Katalysator hergestellt. Die Acetylenbasis ist jedoch nicht an jedem Chemiestandort vorhanden, daher ist eine Alternativsynthese für Vinylether wünschenswert.

Weiterhin kann man Vinylether durch Alkoholabspaltung von Acetalen herstellen. Diese kann rein thermisch, aber auch homogen- bzw. heterogenkatalysiert erfolgen. Die bisher verwendeten Katalysatoren sind teils in ihrer Anwendungsbreite beschränkt, teils in ihrer Aktivität, Selektivität und Standzeit verbesserungsbedürftig.

Es ist bekannt, daß man Vinylether durch Abspaltung von Alkoholen aus Acetalen an Aluminosilikatzeolithen erhalten kann. Diese Zeolithe zeigen in ihrer aciden H-Form unbefriedigende Selektivitäten und Umsätze. Durch Na-Dotierung der Zeolithe ist es nur möglich, die Selektivität bei unvollständigem Umsatz zu steigern.

Es wurde nun gefunden, daß man Vinylether der Formel (I)]

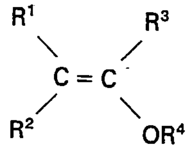

(I)

in der $R^1$ bis $R^3$ untereinander gleich oder verschieden sind und für Wasserstoff, geradkettig oder verzweigte Alkyl- oder Alkenylreste mit bis zu 12 Kohlenstoffatomen, halogensubstituierte Alkylreste, Cycloalkyl- oder Cycloalkenylreste mit 5 bis 8 Kohlenstoffatomen, Aryl-, Alkylaryl-, Alkenylaryl-, Aralkyl- und Aralkenylreste mit 6 bis 16 Kohlenstoffatomen, halogensubstituierte Arylreste oder für heterocyclische Reste stehen und darüber hinaus die Reste $R^1$ und $R^2$ oder $R^1$ und $R^3$ zusammen mit dem C-Atom, an das sie gebunden sind, ein Cycloalkan, Cycloalken oder einen Heterocyclus bilden können, und $R^4$ für Alkyl- oder Alkylaryl- oder Aralkylreste steht in hoher Selektivität bei vollständigem Umsatz aus Acetalen/Ketalen erhält, wenn man Alkohole aus Acetalen/Ketalen der Formel (II)

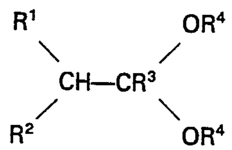

(II)

in der $R^1$ bis $R^4$ obige Bedeutung haben, in Gegenwart von Boro- und/oder Eisensilikatzeolithen als Katalysatoren abspaltet.

Im Hinblick auf den Stand der Technik sind die Ergebnisse des erfindungsgemäßen Verfahrens überraschend, weil die vorangehende Entwicklung gerade in entgegengesetzte Richtung, nämlich die Ausschaltung von aciden Zentren durch die Natriumdotierung, wies. Daher konnte man nicht erwarten, daß mit den Boro- und/oder Eisensilikatzeolithen in der aciden H-Form in so weiten Grenzen so hervorragende Ergebnisse erhalten werden.

Als Reste $R^1$ und $R^3$ kommen unabhängig von $R^4$ Wasserstoff sowie geradkettige oder verzweigte Alkylreste mit 1 bis 12, insbesondere 1 bis 8, vorzugsweise 1 bis 4 Kohlenstoffatomen in Betracht.

Alkyl- oder Alkenylreste sind z.B. Methyl-, Ethyl-, N-Propyl-, i-Propyl-, Propenyl-, i-Propenyl-, n-Butyl-, i-Butyl-, n-Butenyl-, i-Butenyl-, Pentyl-, Pentenyl-, Hexyl-, Hexenyl-, Heptyl-, Heptenyl-, Octyl-, Octenyl-, Nonyl-, Nonenyl-, Decyl-, Decenyl-, Dodecyl- oder Dodecenyl-Reste. Die Alkyl- oder Alkenylreste können noch unter den Reaktionsbedingungen inerte Substitutenten tragen, z.B. Halogen.

Cycloalkylreste sind z.B. Cyclopentyl-, Cyclohexyl-, Cycloheptyl-, Cyclopentenyl- oder Cyclohexenylreste.

Als aromatische Reste kommen z.B. Phenyl-, Benzyl-, 2-Phenylethyl-, 3-Phenylpropyl-, 2-Phenylpropyl-, 4-Phenylbutyl-, 3-Phentylbutyl-, 2-Phenylbutyl- oder 3-Phenylbutenyl-Reste in Betracht, die gegebenenfalls nach durch unter den Reaktionsbedingungen inerte Reste wie Alkylreste oder Halogenatome substituiert sein können.

Als Reste $R^4$ kommen Alkylreste wie Methyl-, Ethyl-, n-/i-Propyl-, Propenyl-, n-, i-, t-Butyl-, Butenyl-, Octyl-, Octenylreste oder Aralkylreste wie Benzyl-, Phenylethyl-, Phenylpropylreste oder Alkylarylreste wie Toluyl-, Xylolreste in Betracht.

Als Ausgangsstoffe sind Acetale von gesättigten aliphatischen Aldehyden z.B. Acet-, Propion- oder Butyraldehyd, Pentanal, Hexanal und höhre homolgene n-Alkanale wie Octanale und Decanale, verzweigte Aldehyde wie Isobutyraldehyd, 2-Methylbutanal, 3-Methylbutanal, 3,3-Dimethylbutanal, 2-Methylpentanal, 2-Ethylhexanal und 2-Methyldecanal geeignet.

Als Ketone für die eingesetzten Ketale kommen beispielsweise die folgenden Verbindungen in Betracht: Methylethylketon, Diethylketon, Methylisopropylketon, Diisopropyl- und Diisobutylketon, Methylisobutylketon, Methylcyclohexanone, Cyclohexanon, Acetophenon und substituierte Acetophenone.

Als Katalysatoren verwendet man Boro- und/oder Eisensilikatzeolithe.

Borosilikatzeolithe kann man bei 90 bis 200°C unter autogenem Druck synthetisieren, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zu Reaktion bringt. Auch gehören hierzu due isotaktischen Zeolithe nach DE—OS 3 006 471 und EP 46 504. Solche Borosilikatzeolithe können ebenefalls hergestellt werden, wenn man die Reaktion statt in wäßriger Aminolösung in etherischer Lösung, z.B. Diethylenglykoldimethylether oder in alkoholischer Lösung, z.B. 1,6-Hexandiol durchführt.

Eisensilikatzeolithe erhält man z.B. aus einer Eisenverbindung, vorzugsweise $Fe_2(SO_4)_3$ und einer Siliciumverbindung, vorzugsweise hochdispersem Soliciumdioxid in wäßriger Aminlösung, insbesondere 1,6-Hexandiamin, mit oder ohne Alkali- oder Erdalkalizusatz bei 100 bis 220°C unter autogenem Druck.

Die so hergestellten Boro- und Eisensilikatzeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110°C/16 h getrocknet und bei 500°C/16 h calciniert.

Man erhält auch gut geeignete Katalysatoren, wenn die isolierten Eisen bzw. Borosilkatzeolithe direkt nach der Trocknung verformt werden und erst nach der Verformung einer Calcinierung unterworfen werden. Die hergestellten Eisen- und Borsilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameinsensäure, Oxalsäure, Essigsäure, Salpetersäure, Ammoniak, Amine, Silikoester und Graphit oder deren Gemische verwendet werden.

Liegen die Zeolithe aufgrund der Art der Herstellung nicht in der katalytisch aktiven, aciden H-Form vor, sondern z.B. in der Na-Form, dann kann man diese durch Ionenaustausch z.B. mit Ammoniumionen und anschließende Calcinierung oder durch Behandlung mit Säuren vollkommen oder partiell in die gewünschte H-Form überführen.

Wenn bei der erfindungsgemäßen Verwendung von zeolithischen Katalysatoren eine durch Koksabschiedung bedingte Desaktivierung eintritt, empfiehlt es sich, die Zeolithe durch Abbrennen der Koksablagerung mit Luft oder mit einem Luft/$N_2$-Gemisch bei 400 bis 550°C, bevorzugt 500°c, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht darin, daß man den umverformten oder verformten Zeolithen mit Metallsalzen durch einen Ionenaustausch oder durch Imprägnierung dotiert. Als Metalle werden Erdalkalimetalle wie Mg, Ca, Sr, Metall der 3., 4., und 5. Hauptgruppe wie Al, Ga, Ge, Sn, Pb, Bi, Übergangsmetalle der 4.—8. Nebengruppe wie Ti, Zr, V, Nb, Cr, Mo, W, Mn, R, Fe, Ru, Os, Co, Rh, Ir, Ni, Pd, Pt, Übergangsmetalle der 1. und 2. Nebengruppe wie Cu, Ag, Zn, Seltene Erdmetalle wie La, Ce, Pr, Nd, Er, Yb und Y eingesetzt.

Zweickmäßigerweise führt man die Dotierung so durch, daß man den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht darin, daß man $Cu(NO_3)_2 \times 3\ H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150°C getrocknet und bei etwa 550°C calciniert. Dieser Trañkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100°C unter Rühren etwa 24 h

aufzuschlämmen. Nach Abfiltrieren, Trocknen bei etwa 150°C und Calcinierung bei etwa 500°C kann das so gewonnene zeolithische Material mit oder ohne Bindemittel zu Strängen Pellets oder Wirbelgut weiterverarbeitet werden.

Ein Ionenaustausch des in der H-Form oder Ammonium-Form oder Alkali-Form vorliegenden Zeolithen kann zo vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material — verformt oder unverformt — einer Behandlung mit Säuren wie Salzsäure, Flußsäure und Phosphorsäure und/oder Wasserdampf unterwirft. Dabei geht man vorteilhaft so vor, daß man Zeolithe in Pulverform mit 1 n Phosphorsäure 1 Stunde bei 80°C behandelt. Nach der Behandlung wird mit Wasser gewaschen, bei 100°C/16 Stunden getrocknet und bei 500°/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 Gew.%igen, insbesondere 12 bis 20 Gew.%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit 0,001 n bis 2 n Flußsäure, vorzugsweise 0,05 n bis 0,5 n Flußsäure, behandelt, beispielsweise durch Erhitzen unter Rückflüß über einen Zeitraum von 0,5 bis 5, vorzugsweise 1 bis 3 Stunden. Nach Isolierung, z.B. durch Abfiltrieren und Auswaschen, des zeolithischen Materials wird dieses zweckmäßig, bei Temperaturen von 100 bis 160°C, getrocknet und bei Temperaturen von 450°C bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50°C bis 90°C, vorzugsweise 60°C bis 80°C, über einen Zeitraum von 0,5 bis 5, vorzugsweise mit 12 bis 20 Gew.%iger Salzsäure, behandelt. Anschließend wird das zeolithische Material ausgewaschen und zweckmäßig bei Temperaturen von 100 bis 160°C getrocknet und bei 450 bis 600° calciniert. Einer HF-Behandlung kann sich auch eine HCl-Behandlung anschließen.

Nach einer anderen Arbeitsweise kann man Zeolithe durch Aufbringung von Phosphorverbindungen, wie Trimethoxiphosphat, Trimethoxiphosphin, primären, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110°C getrocknet und bei 500°C calciniert.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Für das erfindungsgemäße Verfahren werden folgende Reaktionsbedingungen gewählt.

Die Umsetzung führt man vorteilhaft in der Gasphase bei Temperaturen von 100 bis 500°C, insbesondere von 150 bis 350°C, und bei einem Druck von 0,1 bis 100 bar, insbesondere 0,5 bis 10 bar durch. Bei der Umsetzung in der Gasphase hält man vorteilhaft eine Katalysatorbelastung von 0,1 bis 20, insbesondere von 1 bis 10 g Ausgangstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden. Es ist auch möglich, die Reaktion in der Flüssigphase (Suspension-, Riesel- oder Sumpffahrweise) bei Temperaturen zwischen 50 und 200°C auszuführen. Die Durchführung der Reaktion kann diskontinuierlich, vorzugsweise kontinuierlich erfolgen. Besonders vorteilhaft kann die Anwendung von vermindertem Druck sein.

Schwerflüchtige oder feste Ausgangsstoffe werden in gelöster Form, z.B. in THF-Toluol- oder Petrolether-Lösung zum Einsatz gebracht. Generell ist eine Verbündung des Eduktes mit derartigen Lösungsmitteln oder mit Inertgasen wir $N_2$, Ar, $H_2O$-Dampf möglich.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bei dieser Arbeitsweise werden gasförmigen Reaktionsprodukte sofort in eine Trennung eingebracht und z.B. in einer Fraktionierkolonne in ihre Einzelkomponenten zerlegt. Eine bevorzugte Ausführungsform besteht darin, daß man die Reaktionsausträge in einer wäßrigen Hydrogencarbonat-Lösung z.B. $KHCO_3$ oder $NaHCO_3/Na_2SO_4$ quencht.

## Beispiele 1—16

Die Reaktionen in der Gasphase werden unter isothermischen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch nach bekannter Methode.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexadiamin-Lösung (Mischung 50:50 Gew.%) bei 170°C unter autogenem Druck in einem Rührautoklaven hergestellt. Nach Abfiltrieren und Auswaschen wird des kristalline Reaktionsprodukt bei 100°C/24 h getrocknet und bei 500°C/24 h calciniert. Diese Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2-mm-Stränge hergestellt, die bei 110°C/16 h getrocknet und bei 500°/24 h calciniert werden.

Katalysator B

Der Eisensilikatzeolith des Pentasil-Typs wird unter hydrothermalen Bedingungen bei autogenem Druck und 165°C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelfsäure und 425 g Wasser in einem Rührautoklaven während 4 Tagen synthetisiert. Der Zeolith wird abfiltriert, ausgewaschen, bei 110°C/24 g getrocknet und bei 500°/24 h calciniert. Man erhält einen Eisensilikatzeolithen mit einem $SiO_2/Fe_2O_3$-Verhältnis von 17,7 und einen $Na_2O$-Gehalt von 1,2 Gew.%. Dieser Zeolith wird mit hochdispersem $SiO_2$ im Gewichtsverhältnis 80:20 zu 2,5-mm-Strängen verstrangt, bei 110°C/16 h getrocknet und bei 500°C/24 h calciniert.

Katalysator C

Katalysator A wird mit wäßriger $Ce(NO_3)_3$-Lösung imprägniert, bei 130°C/2 h getrocknet und bei 540°C/2 h calciniert. Der Ce-Gehalt beträgt 1,8 Gew.%.

Die Versuchsergebnisse und die Reaktionsbedingungen sind in der folgenden Tabelle zusammengestellt.

Reaktion A) 1,1-Dimethoxyethan → Methylvinylether + Methanol

Reaktion B) 1,1-Dimethoxy-1-methylpropan → 1-Methoxy-2-methyl-prop-1-en + Methanol

Reaktion C) 1,1-Di-Propoxy-butan → 1-i-Propoxy-but-1-en + Isopropanol mit THF 75;25 verdünnt

Reaktion D) 1,1-Dimethoxy-octan → 1-Dimethoxyoct-1-en + Methanol mit THF 75;25 verdünnt

Reaktion E) 1,1-Dimethoxy-2-phenylethan → 1-Methoxystyrol + Methanol mit THF 50:50 verdünnt

Reaktion F) 1,1-Dimethoxy-2-[p-fluorophenyl]ethan → 1-Methoxy-p-fluorstyrol + Methanol mit THF 50:50 verdünnt

Reaktion G) 1-Phenyl-1,1-dimethoxyethan → 1-Phenyl-1-methoxyethan + Methanol mit THF 50:50 verdünnt

Reaktion H) Cyclohexanondiethylketal → Cyclohexenylethylether + Ethanol mit THF 50:50 verdünnt

Verdünnung ist in Gew.% angegeben.

Beispiel 17

Es werden 200 ml/h 98 %iges 1,1-Dimethoxyoctan in einem Stickstoffstrom von 300 ml/h verdampft und bei einer Temperatur von 300°C über 1 l des Borzeolithkatalysators A, der in einem von außen elektrisch beheizten Reaktionsrohr untergebracht ist, geleitet.

Das gasförmige Reaktionsgemisch wird in den Mittelteil einer Fraktionierkolonne geleitet, das gebildete Methanol sowie andere Leichtsieder destillieren über Kopf ab, während das 1-Methoxioct-1-en im Sumpf der Kolonne abgezogen werden kann.

Die Reinherstellung gelingt in einfacher Weise durch eine übliche Destillation.

Der Umsatz von 1,1-Dimethoxyoctan ist vollständig, Ausbeute beträgt 94,4% d. Th.

Der Katalysator A zeigt nach 120 Stunden Reaktionszeit noch keine Desaktivierungserscheinungen.

Beispiel 18

Es wird wir im Beispiel 17 verfahren, jedoch 80 ml/h einer 50 %igen THF 1,1-Dimethoxy-2-phenylethan-Lösung zur Reaktion bei 230°C gebracht.

Die Ausbeute an Methylstyrolether beträgt 93,9% d. Th. bei einem Umsatz von 100%.

Beispiel 19

Es wird wie im Beispiel 17 verfahren, jedoch 80 ml/h einer 50 %igen THF/Hexyl-dimethoxymethan-Lösung bei 230°C zur Reaktion gebracht.

Die Ausbeute an Cyclohexanmethylenmethylether beträgt 85,5% d. Th. bei 100% Umsatz.

Beispiel 20

Es wird wie im Beispiel 17 verfahren, jedoch werden 120 ml/h einer 50 %igen THF 1,1-Diethoxy-3-chlorpropan-Lösung bei 240°C zur Reaktion gebracht.

Die Ausbeute an 1-Methoxy-3-chlorprop-1-en beträgt 70,5% d. Th. bei einem Umsatz von 100%.

TABELLE 1

| Beispiel | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Reaktion | A) | A) | B) | B) | B) | C) | C) | D) | D) | E) | E) | E) | F) | F) | G) | H) |
| Katalysator | A | B | A | C | B | A | B | A | C | A | A | B | A | C | A | A |
| Temperatur °C | 300 | 300 | 300 | 300 | 300 | 300 | 300 | 300 | 300 | 250 | 300 | 300 | 300 | 300 | 300 | 300 |
| WHSV $h^{-1}$ | 2 | 2 | 2 | 2 | 2 | 2 | 2 | 2 | 2 | 3 | 3 | 3 | 3 | 3 | 3 | 3 |
| Umsatz (II) % | 100 | 100 | 100 | 100 | 100 | 100 | 100 | 100 | 100 | 100 | 100 | 100 | 100 | 100 | 100 | 100 |
| Selektivität (I) % | 95,6 | 93,7 | 92,8 | 93,5 | 89,7 | 92,2 | 87,7 | 94,2 | 92,6 | 95,4 | 92,4 | 88,7 | 90,1 | 92,7 | 95,7 | 87,2 |

**Patentansprüche**

1. Verfahren zur Herstellung von Vinylethern der Formel (I)

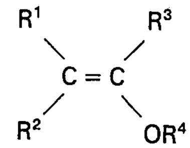

(I)

in der $R^1$ bis $R^3$ untereinander gleich oder verschieden sind und für Wasserstoff, geradkettig oder verzweigte Alkyl- oder Alkenylreste mit bis zu 12 Kohlenstoffatomen, halogensubstituierte Alkylreste, Cycloalkyl- oder Cycloalkenylreste mit 5 bis 8 Kohlenstoffatomen, Aryl-, Alkylaryl-, Alkenylaryl-, Aralkyl- und Aralkenylreste mit 6 bis 16 Kohlenstoffatomen, halogensubstituierte Arylreste oder für heterocyclische Reste stehen und darüber hinaus die Reste $R^1$ und $R^2$ oder $R^1$ und $R^3$ zusammen mit dem C-Atom, an das sie gebunden sind, ein Cycloalkan, Cycloalken oder einen Heterocyclus bilden können, und $R^4$ für Alkyl- oder Alkylaryl- oder Aralkylreste steht, man Alkohole aus Acetalen/Ketalen der Formel (II)

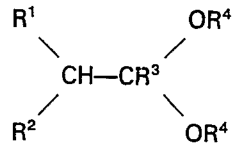

(II)

in der $R^1$ bis $R^4$ obige Bedeutung haben, in Gegenwart von Boro- und/oder Eisensilikatzeolithen als Katalysatoren abspaltet.

2. Verfahren nach Anspruch 1, dadurch gekennzeichnet, daß man die Boro- und/oder Eisensilikatzeolithe verwendet, die aus wäßrigen Polyamin-Lösungen synthetisiert worden sind.

3. Verfahren nach den Ansprüchen 1 und 2, dadurch gekennzeichnet, daß man die Umsetzung in der Gasphase durchführt.

**Revendications**

1. Procédé de préparation d'éthers vinyliques de formule (I)

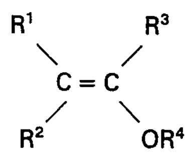

(I)

dans laquelle $R^1$ à $R^3$ sont identiques ou différents et sont mis pour des atomes d'hydrogène, des restes alkyle ou alcényle linéaires ou ramifiés ayant jusqu'à 12 atomes de carbone, des restes halogénoalkyle, des restes cycloalkyle ou cycloalcényle avec de 5 à 8 atomes de carbone, des restes aryle, alkylaryle, alcénylaryle, aralkyle ou aralcényle avec de 5 à 16 atomes de carbone ou des restes hétérocycliques, et en outre les restes $R^1$ et $R^2$ ou $R^1$ et $R^3$ peuvent former avec l'atome de carbone auquel ils sont liés un cycloalcane, un cycloalcène ou un hétérocycle, et $R^4$ est mis pour un reste alkyle, alkylaryle ou aralkyle, caractérisé en ce qu'on élimine des alcools à partir d'acétals/cétals de formule II

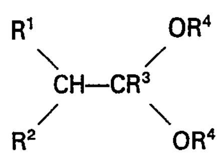

(II)

dans laquelle $R^1$ à $R^4$ ont les significations ci-dessus, en présence de zéolithes de boro- et/ou ferrosilicate en tant que catalyseurs.

2. Procédé selon la revendication 1, caractérisé en ce qu'on utilise des zéolithes de boro- et/ou ferrosilicate qui ont été synthétisées à partir de solutions aqueuses de polyamines.

3. Procédé selon les revendications 1 ou 2, caractérisé en ce qu'on effectue la réaction en phase gazeuse.

**Claims**

1. A process for the preparation of the vinyl ether of the formula (I)

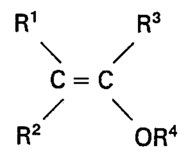

(I)

where $R^1$ to $R^3$ are identical or different and are each hydrogen, a straight-chain or branched alkyl or alkenyl radical of not more than 12 carbon atoms, halogen-substituted alkyl, a cycloalkyl or cycloalkenyl radical of 5 to 8 carbon atoms, an aryl, alkylaryl, alkenylaryl, aralkyl or aralkenyl radical of 6 to 16 carbon atoms, halogen-substituted or a heterocyclic radical and furthermore $R^1$ and $R^2$ or $R^1$ and $R^3$, together with the carbon atoms to which they are bonded, may form a cycloalkane, cycloalkene or heterocyclic structure, and $R^4$ is alkyl, alkylaryl or aralkyl, wherein an alcohol is eliminated from an acetal or ketal of the formula (II)

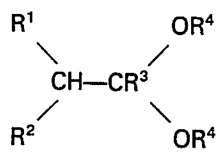

(II)

where $R^1$ to $R^4$ have the above meanings, in the presence of borosilicate and/or iron silicate zeolites as catalysts.

2. A process as claimed in claim 1, wherein the borosilicate and/or iron silicate zeolites synthesized from aqueous polyamine solution are used.

3. A process as claimed in claims 1 and 2, wherein the reaction is carried out in the gas phase.